# EUROPÄISCHE PATENTSCHRIFT

(11) **EP 3 147 266 B1**
(45) Veröffentlichungstag und Bekanntmachung des Hinweises auf die Patenterteilung: **06.11.2024**
(21) Anmeldenummer: 16187410.2
(22) Anmeldetag: 06.09.2016
(51) Int. Cl.: C03C 3/093, C03C 4/00, C03C 8/04, A61L 27/00, A61F 2/00

(54) **MEDIZINISCHES GLASELEMENT**
MEDICAL GLASS ELEMENT
ÉLEMENT EN VERRE MEDICAL

(30) Priorität: 22.09.2015 DE 102015115958
(43) Veröffentlichungstag der Anmeldung: 29.03.2017
(73) Patentinhaber: SCHOTT AG, 55122 Mainz (DE)
(72) Erfinder: PICHLER-WILHELM, Sabine, 84028 Landshut (DE); LAUTENSCHLÄGER, Christian, Cambridge, Cambridgeshire CB4 8HN (GB); MAKAREWICZ, Oliwia, 07443 Jena (DE); KLINGER-STROBEL, Mareike, 07443 Jena (DE); STALLMACH, Andreas, 06648 Eckartsberga (DE); PLETZ, Mathias W. R., 31535 Neustand am Rübenberge (DE)
(74) Vertreter: Blumbach · Zinngrebe Patentanwälte PartG mbB

(56) Entgegenhaltungen:
- WO-A2-2004/076369
- DE-A1- 10 120 475
- DE-A1- 102004 023 732
- US-A1- 2007 172 661
- US-A1- 2012 219 792

## Beschreibung

Die Erfindung betrifft ein in den menschlichen oder tierischen Körper einbringbares Element, das zumindest bereichsweise ein Glas aufweist.

Biofilme sind komplexe Gemeinschaften von Mikroorganismen in nährstoffreichen wässrigen Systemen. Ihre Bildung hängt zusammen mit der Adhäsion und Kolonisierung von Oberflächen durch die Produktion von klebrigen extrazellulären Polymeren. Die Bildung von Biofilmen wird auch als Mikrofouling bezeichnet und ist der erste Schritt des Makrofoulings, das heißt der Besiedlung beziehungsweise Entwicklung von makroskopischen Foulingorganismen.

Im medizinischen Bereich sind dauerhaft im beziehungsweise am Körper vorhandene Bauteile wie Katheter, Prothesen, künstliche Herzklappen, Implantate, Herzschrittmacher und so weiter äußerst anfällig für Mikrofouling. Damit geht die Gefahr Biofilm-assoziierter Erkrankungen und Infektionen einher.

Biofilme sind weitgehend unempfindlich gegen Umweltfaktoren wie ultraviolette Strahlung und gegen chemische Behandlungen mit zum Beispiel Detergentien, also grenzflächenaktiven Stoffen. Zudem sprechen Biofilme kaum auf Antibiotika an und verfügen über einen Schutz gegenüber der Immunabwehr des Wirts. Es wird geschätzt, dass *S*. *aureus* und *S*. *epidermidis* ungefähr 40 % bis 50 % der Infektionen an künstlichen Herzklappen und zwischen etwa 50 % und 70 % der Biofilminfektionen an Kathetern verursachen.

Die US-Patentanmeldung US 2012/0219792 A1 betrifft ein Glassubstrat für ein Displaycover mit ausgezeichneter Festigkeit, antibakteriellen Eigenschaften hoher Transparenz und hoher Transmission im Bereich des sichtbaren Lichts.

Die internationale Patentanmeldung WO 2004/076369 A1 beschreibt ein antimikrobiell wirkendes Borosilicatglas.

Die DE 101 20 475 A1 beschreibt ein Borosilicatglas mit geringer Verarbeitungstemperatur.

Die DE 10 2004 023 732 A1 beschreibt Verwendungen von siliciumhaltigen und phosphorfreien Gläsern zur Behandlung entzündlicher Erkrankungen.

Schließlich betrifft die US-Patentanmeldung US 2007/0172661 A1 antimikrobielle Glas- und Glaskeramikoberflächen und ihre Herstellung.

Es ist eine Aufgabe der Erfindung, einen Werkstoff bereitzustellen, der von Biofilmen weniger besiedelt wird als bekannte Werkstoffe für in den menschlichen oder tierischen Körper anbringbare oder in den Körper einbringbare Elemente.

Es ist eine weitere Aufgabe der Erfindung, einen Werkstoff bereitzustellen, welcher darüber hinaus für den Körper gut verträglich ist.

Diese Aufgaben werden in höchst überraschender Weise für den Fachmann bereits gelöst mit einem in den menschlichen oder tierischen Körper einbringbaren Element, wobei das Element aus der Gruppe ausgewählt ist, welche Gehäuse für elektronischen Geräte wie Transponder, Defibrillatoren, Herzschrittmacher umfasst und das zumindest bereichsweise ein Glas aufweist, welches die Bildung von Biofilmen hemmt und/oder nicht cytoxisch für menschliche oder tierische Zellen und blutverträglich ist, wenn das Element in den menschlichen oder tierischen Körper eingebracht oder an diesen angebracht ist, wobei das Glas zumindest

| | |
|---|---|
| SiO₂ | im Bereich von 60 bis 75 Gew.-% und |
| ZnO | im Bereich von 1 bis 7 Gew.-% |

umfasst, wobei das Element zumindest ein Gehäuse umfasst oder ein Gehäuse ist, welches für die Aufnahme elektronischer Bauteile ausgebildet ist, wobei das Gehäuse zumindest bereichsweise aus dem Glas besteht und/oder wobei das Gehäuse zumindest bereichsweise zumindest eine Schicht aus dem Glas aufweist.

Vorteilhaft ist es, wenn das Element die Bildung von Biofilmen hemmt. Alternativ ist es möglich und ebenso vorteilhaft, dass menschliche oder tierische Zellen an das Element anwachsen, wenn es in den menschlichen oder tierischen Körper eingebracht oder an diesen angebracht ist.

Besonders vorteilhaft ist es, wenn das Element sowohl die Bildung von Biofilmen hemmt als auch wenn menschliche oder tierische Zellen an das Element anwachsen, wenn es in den menschlichen oder tierischen Körper eingebracht oder an diesen angebracht ist. All diese Eigenschaften sind von der Erfindung umfasst.

Die Erfinder haben herausgefunden, dass überraschend einerseits das Glas die Bildung von Biofilmen hemmt, und vorteilhaft die menschlichen oder tierischen Zellen an das Glas anwachsen, wenn das Element in den menschlichen oder tierischen Körper eingebracht ist. Eukaryotische und prokaryotische Zellen reagieren nach Erkenntnissen der Erfinder in entgegengesetzter Weise auf das Glas mit der erfindungsgemäßen Zusammensetzung. Dies nutzt die Erfindung durch die Anwendung des Glases für Bauteile, die mit dem menschlichen oder tierischen Körper in Berührung kommen, insbesondere sogar in diesen eingebracht werden, bei denen hohe Hygieneanforderungen bestehen. Dasselbe Material ermöglicht gemäß der Erfindung zum Einen die Hemmung der Anlagerung von Biofilmen und bevorzugt ebenso die Verträglichkeit bis hin zum Ein- beziehungsweise Anwachsen in (an) die Körperzellen. Es hat sich gezeigt, dass eukaryotische Zellen bezüglich ihres Wachstum und der normalen Zellteilung nicht beeinflusst werden (d.h. keine besondere Zellhaftung bei eukaryotischen Zellen). Die erfindungsgemäßen Materialien sind damit nicht zytotoxisch.

Im Rahmen der Anmeldung ist die Angabe "Gew.-%" auf die Gesamtmenge der Oxide bezogen, die Mengenangaben der Komponenten des Glases sind also in "Gew.-% auf Oxidbasis" angegeben.

"Element" ist ein Körper, der aus Vollmaterial bestehen kann, jedoch auch mit zumindest einem Hohlraum ausgebildet sein kann. Die Formgebung für das erfindungsgemäße Element ist im Rahmen der Fertigungsmöglichkeiten für Gegenstände aus Glas für den Fachmann je nach Einsatzgebiet für das Element frei wählbar.

Die weiteren konstituierenden Komponenten bzw. Oxide des Glases wird der Fachmann für den jeweils betreffenden Anwendungsfall basierend auf seinem Können und auch im Hinblick auf die spätere Anwendung, die Verbindung mit anderen Werkstoffen (z.B. Metalle oder Keramiken) , die Art des Einschmelzens und/oder der Weiterverarbeitung auswählen. Zudem kann die Zugabe von Läutermitteln erfolgen.

"Anwachsen menschlicher oder tierischer Zellen" bedeutet, dass das Glas nicht cytotoxisch für diese Zellen und blutverträglich (hämokompatibel) ist. Die Wechselwirkung zwischen der Oberfläche des Glases mit Erythrocyten als den Hauptkomponenten des Bluts und einem der ersten Kontaktpartner nach systemischer Administration wurden anhand der Hämolyse und der Erythrocytenaggregation bestimmt.

Die Cytotoxizität wurde von den Erfindern in Bezug auf Muskelzellen, Caco2-Zellen und L929-Maus-Fibroblasten bestimmt. Dazu wurde das Glas mit den jeweiligen Zellen in DMEM (Life technologies) für 48 Stunden kultiviert. Die Versuche wurden bei 37 °C und einer Atmosphäre von + 5% CO2 durchgeführt. Dies entspricht standardisierten Tests zur Zytotoxizität auf eukaryotischen Zellen. Die Cytotoxizität wurde anhand des Zellwachstums und der Zellmorphologie evaluiert. Morphologische Veränderungen und Zellbeschädigungen wurden qualitativ anhand digitaler Fotografien durch ein Lichtphasenkontrastmikroskop ermittelt.

Zur Bestimmung der Hämolyse und der Erythrocytenaggregation wurden menschliche rote Blutkörperchen für zwei Stunden bei 37°C auf dem betreffenden Glas inkubiert. Die roten Blutkörperchen wurden gewonnen aus Blut, welches in heparinisierten Röhrchen gesammelt wurde. Die Suspensionen der roten Blutkörperchen wurden dann innerhalb von 24 Stunden verbraucht.

Die Freisetzung von Hämoglobin wurde herangezogen, um einen beschädigenden Effekt des Glases und/oder glaskeramischen Materials auf die Erythrocytenmembran zu quantifizieren. Die Behandlung der roten Blutkörperchen mit PBS-Puffer und einer Lösung von 1% Triton X-100 führten zu 0% beziehungsweise 100 % Hämolyse. Zur Bestimmung der Hämolyse wurde die Hämoglobinausschüttung durch spektroskopische Analyse des Überstandes bei 544 nm bestimmt. Eine Freisetzung von 0 bis 2 % der Gesamtfreisetzung wurde als nicht-hämolytisch, eine Freisetzung im Bereich zwischen 2 % und 5 % der Gesamtfreisetzung als leicht hämolytisch und eine Freisetzung von mehr als 5 % der Gesamtfreisetzung wurde als hämolytisch gemäß dem ASTM F756-08 Standard eingestuft.

Zur Bestimmung der Erythrocytenaggregation wurden mikroskopische Aufnahmen durch Klassifizierung in drei Stufen bewertet. In Stufe 1 blieben die roten Blutkörperchen diskret in der Suspension verteilt, ohne dass eine Aggregation detektierbar ist. In Stufe 2 zeigt sich eine moderate Aggregation mit der Bildung von Rouleaux, wobei jedoch die Mehrheit der Erythrocyten diskret vorliegt. Dies wurde in mikroskopischen Beobachtungen festgestellt, deren Methodik bekannt ist (siehe Bauer et al, Macromol. Biosci. 2012, 12, 986-998). In Stufe 3 sind nahezu alle roten Blutkörperchen zu Clustern aggregiert. Zur Bestimmung von Stufe 1 wurden die Erythrocyten mit PBS als negative Kontrolle behandelt. Positive Kontrollen wurden mit 30 mg/mL 25kDa verzweigtem Polyethylenimin behandelt und als Stufe 3 klassifiziert.

Der Ausdruck "Hemmen der Bildung von Biofilmen" bedeutet, dass im Vergleich zu üblicherweise als Substrat für mikrobielle Besiedlung verwendeten Gläsern und/oder Glaskeramiken wie Borosilikatglas - beispielsweise "Borofloat 33" der Anmelderin - oder Kalk-Natron-Glas (Paul Marienfeld GmbH) eine geringere Fläche von Biofilm bedeckt wird, wenn das erfindungsgemäße Glas verwendet wird.

Die Bedeckung mit Biofilm kann beispielsweise durch Visualisierung bestimmt werden. Die Erfinder führten entsprechende Experimente durch mit Hilfe eines LSM510 konfokalen Laserscanning-Mikroskops durch Rastern einer Fläche von 100 mm (Mikrometer) auf 100 mm (Mikrometer) auf dem grünen Kanal (480/500 nm) und dem roten Kanal (490/635 nm). Dabei wurde ein 40 x 10 Wasserimmersionsobjektiv benutzt. Die Aufnahmen der Biofilme wurden mittels der ZEN 9.0 software (Carl Zeiss Microscopy GmbH) sichtbar gemacht.

Die Fläche, welche innerhalb einer Aufnahme (image) von Biofilm bedeckt ist, wird dabei berechnet basierend auf der Trennung von Pixeln vom Hintergrund je nach ihrer Helligkeit. Ein 16-bit-lmage wird dabei in 256 Graustufen (0 = schwarz; 255 = weiß) aufgeteilt. Durch Ansetzen eines Schwellenwertes nach Otsu können so alle bedeckten Gebiete zuverlässig identifiziert werden. Die von Biofilm bedeckte Fläche wurde in Relation zur Gesamtzahl an Pixeln in einer Aufnahme mittels der Software imageJ 1.43u berechnet.

Unter einem "glaskeramischen Material" wird ein Werkstoff verstanden, welcher aus denselben Ausgangsmaterialien wie ein Glas hergestellt wird, wobei jedoch bei der Verarbeitung vor, während oder im Anschluss an die Formgebung das Glas beispielsweise durch gezielte Temperaturführung in einen teils kristallinen und teils amorphen, also glasartigen Zustand überführt wird. Auch die vollständige Entglasung ist dabei möglich.

Als glaskeramisches Material wird üblicherweise ein Material verstanden, in dem sowohl kristalline Phasen und amorphe Phase, d.h. Glasphasen, gleichzeitig bestehen. Insbesondere können Bereiche mit kristallinen Phasen durch Glasphasen miteinander verbunden sein. Eine Glaskeramik kann sozusagen als teilweise kristallisiertes Glas aufgefasst werden, wobei der Kristallisationsgrad sehr weit variieren kann, sogar bis nahe an 100%.

In einer vorteilhaften Weiterbildung der Erfindung umfasst das Glas zumindest die folgenden Komponenten

| | |
|---|---|
| SiO₂ | im Bereich von 60 - 75 Gew.-% |
| R₂O | im Bereich von 5 bis 20 Gew.-% |
| RO | im Bereich von 0 bis 10 Gew.-% |
| Al₂O₃ | im Bereich von 1 bis 6 Gew.-% |
| B₂O₃ | im Bereich von 0 bis 10 Gew.-% |
| TiO₂ | im Bereich von 0 bis 8 Gew.-% |
| ZnO | 1 bis 7 Gew.-% |
| FeO | 0 bis 5 Gew.-% |

wobei R₂O ein Oxid oder eine Kombination von Oxiden ist ausgewählt aus der Gruppe, welche Li₂O, Na₂O und K₂O umfasst, und wobei RO ein Oxid oder eine Kombination von Oxiden ist ausgewählt aus der Gruppe, welche MgO und/oder CaO und/oder BaO und/oder SrO umfasst. Die Gesamtmenge von R₂O und/oder RO kann von einem oder mehreren Oxiden der jeweils angegebenen Gruppe gebildet werden, in beliebiger Kombination der Oxide.

Eisenoxid wird dem Glas zugesetzt um eine Verschmelzung mittels IR-Strahlern oder Laser bei einer Wellenlänge von 1060nm durchzuführen. Die Absorption soll dabei bevorzugt zwischen 2 und 20% liegen. Dies ist bei FeO Gehalten von 2,7 bis 5 % der Fall. Je dünner die Wandstärke des zu verschmelzenden Glases ist, desto stärker soll die Absorption und damit verbunden desto höher der FeO-Gehalt sein. Falls beim Erschmelzen des Glases das Eisen in Form von Fe₂O₃ zugegeben wird, ist durch eine geeignete Schmelzführung sicherzustellen, dass eine ausreichende Menge des Fe (III) zu Fe (II) reduziert wird, um die genannte FeO Anteile im Glas zu erhalten. Es kann im Glas in unterschiedlichen Oxidationszuständen vorliegen und wird hier als FeO angegeben. Der Fachmann ist damit vertraut, dies in Anteile von Fe (III) und Fe₂O₃ umzurechnen, wenn er z.B. zu Synthesezwecken den Anteil von Fe₂O₃ angeben möchte.

Insbesondere hat es sich als vorteilhaft herausgestellt, wenn das Glas

| | |
|---|---|
| SiO₂ | im Bereich von 60 bis 70 Gew.-% |
| R₂O | im Bereich von 5 bis 20 Gew.-% |
| RO | im Bereich von 0 bis 10 Gew.-% |
| Al₂O₃ | im Bereich von 1 bis 5 Gew.-% |
| B₂O₃ | im Bereich von 0 bis 10 Gew.-% |
| TiO₂ | im Bereich von 0 bis 8 Gew.-% |
| ZnO | im Bereich von 1 bis 7 Gew.-% |
| FeO | im Bereich von 0 bis 5 Gew.-% |
| mit | |
| Na₂O | im Bereich von 2 bis 10 Gew.-% und/oder |
| K₂O | im Bereich von 3,5 bis 10 Gew.-%. |

umfasst, wobei R₂O ein Oxid oder eine Kombination von Oxiden ist ausgewählt aus der Gruppe, welche Li₂O, Na₂O und K₂O umfasst, und wobei RO ein Oxid oder eine Kombination von Oxiden ist ausgewählt aus der Gruppe, welche MgO und/oder CaO und/oder BaO und/oder SrO umfasst.

Besonders vorteilhaft ist es, wenn das Glas bis auf höchstens Verunreinigungen frei ist von CuO. Verunreinigungen können in der Regel bis zu einem Gehalt von 0,3% vorhanden sein. Dies bedeutet, dass der CuO-Gehalt vorteilhaft 0 bis 0,3% beträgt, ganz besonders vorteilhaft sind 0%.

Das beschriebene Glas ist zudem insbesondere geeignet, um hermetische Durchführungen, insbesondere mit Titan und/oder verschiedenen Titanlegierungen herzustellen. In einer solchen Durchführung wird üblicherweise ein Funktionselement durch ein Isolationselement in einer Durchführungsöffnung eines Trägerelements gehalten, wodurch die Durchgangsöffnung hermetisch verschlossen wird. Das Trägerelement kann insbesondere ein Gehäuse und/oder ein Gehäuseteil sein, hier insbesondere eines Gehäuses oder Gehäusebauteils aus Titan oder Titanlegierungen, und das Funktionselement kann insbesondere ein Leiter für elektrischen Strom sein. Andere Funktionselemente, wie z.B. Lichtleiter, Wellenleiter, Hohlleiter, Röhrchen, Kapillare usw. sind natürlich ebenso von der Erfindung umfasst. Das Isolationselement wird aus dem beschriebenen hergestellt und liegt beispielsweise in Form eines Pfropfens vor.

Die Eignung des Glases insbesondere für die Herstellung von Durchführungen mit Titan und/oder Titanlegierungen ist durch dessen chemische Kompatibilität mit diesen Metallen und/oder dessen thermischen Ausdehnungskoeffizienten begründet. Besonders vorteilhaft ist der thermische Ausdehnungskoeffizient des Glases niedriger als derjenige von Titan und/oder der betreffenden Titanlegierung, so dass eine sogenannte Druckdurchführung hergestellt werden kann. In einer solchen schrumpft beim Einschmelzen des Funktionselements mit Hilfe des Isolationsmaterials in das Trägerelement das Trägerelement auf das Isolationselement aufschrumpfen, so dass das Trägerelement zumindest bei Raumtemperatur eine Druckspannung auf das Isolationselement ausübt. Dadurch kann insbesondere die Auszugskraft, d.h. die Kraft, die benötigt wird, um das Isolationselement aus dem Trägerelement herauszudrücken, signifikant erhöht werden und/oder eine zuverlässig und Dauerhaft hermetisch dichte Durchführung zur Verfügung gestellt werden.

Besonders gute Resultate wurden erzielt mit einem, welches

| | |
|---|---|
| SiO₂ | im Bereich von 62 bis 66 Gew.-% |
| Al₂O₃ | im Bereich von 3,8 bis 4,5 Gew.-% |
| B₂O₃ | im Bereich von 0 bis 10 Gew.-% |
| TiO₂ | im Bereich von 3,5 bis 4,5 Gew.-% |
| ZnO | im Bereich von 5 bis 7 Gew.-% |
| FeO | im Bereich von 0,001 bis 0,005 Gew.-% |
| Na₂O | im Bereich von 5,9 bis 6,5 Gew.-% und |
| K₂O | im Bereich von 8,3 bis 9,1 Gew.-% |

enthält. Dabei können bis zu 0,04 Gew.-% SeO₂ vorhanden sein.

Insbesondere kann das Glas
einen Anteil von Ag von weniger als 0,3 Gew.-%. und/oder
einen Anteil von P₂O₅ von weniger als 0,5 Gew.-%. und/oder
einen Anteil von F von weniger als 1 Gew.-%.
umfassen. Besonders vorteilhaft ist es, wenn der Anteil von Ag und/oder P₂O₅ möglichst gering gewählt wird, insbesondere wenn das Glas bis auf höchstens Verunreinigungen frei von diesen Stoffen ist. Dies umfasst dann insbesondere auch den Anteil von 0%. Diese Untergrenze gilt ebenso für F_{2.} Es kann aber ebenso vorteilhaft sein, wenn ein gewisser kleiner Anteil von F₂ in dem Glas vorhanden sind. Daher sieht eine vorteilhafte Ausführungsform einen Anteil von F₂ von mehr als 0 Gew.-% bis zu weniger als 1 Gew.-% vor.

Für die Anwendungen im Körper ist gemäß der Erfindung vorgesehen, dass das Element zumindest ein Gehäuse umfasst oder ein Gehäuse ist, welches für die Aufnahme aktiver und/oder passiver elektronischer Bauteile ausgebildet ist, wobei das Gehäuse zumindest bereichsweise aus dem Glas und/oder glaskeramischen Material besteht und/oder wobei auf das Gehäuse zumindest bereichsweise zumindest eine Schicht aus dem Glas und/oder glaskeramischen Material aufweist. Ebenso möglich ist es, dass zusätzlich das Gehäuse mit Mitteln zum Verabreichen von Wirkstoffen versehen ist, beispielsweise einer Membran und/oder einem Ventil usw.. Auf diese Weise können Wirkstoffe, insbesondere Medikamente, im Körper und vorteilhaft an definierter Stelle abgegeben werden, z.B. direkt in der Blutbahn.

Das erfindungsgemäße Element beinhaltet demnach in einer Ausführungsform ein Gehäuse von elektronischen Bauteilen oder repräsentiert ein solches, welches zumindest bereichsweise von dem Glas gebildet wird oder auf welchem das Glas zumindest bereichsweise als Schicht aufgebracht ist.

In einer besonders einfachen Ausgestaltung umfasst das Gehäuse ein Rohr aus dem Glas. Insbesondere ist das Element ein insbesondere ein- oder beidseitig verschlossenes Rohr aus dem Glas. Das Element ist beispielsweise als ein Gehäuse für elektronische Geräte wie Transponder oder ganze Defibrillatoren, Herzschrittmacher oder Labs-on-Chip oder Kontaktlinse ausgebildet.

Die Offenbarung stellt des Weiteren ein Glas und/oder glaskeramisches und/oder keramisches Material zur Verwendung als die Bildung von Biofilmen hemmendes und/oder antibakterielles Element und/oder die Bildung von Biofilmen hemmender und/oder antibakterieller Bereich eines Elements und/oder die Bildung von Biofilmen hemmende und/oder antibakterielle Schicht auf oder in einem Element, das in den menschlichen oder tierischen Körper einbringbar oder an diesen anbringbar ist, zur Verfügung, wobei das Glas und/oder glaskeramische und/oder keramische Material

| | |
|---|---|
| SiO₂ | im Bereich von 60 bis 75 Gew.-% und |
| ZnO | im Bereich von 1 bis 7 Gew.-% |

umfasst. Dabei handelt es sich insbesondere um ein oben beschriebenes Element.

Außerdem betrifft die Offenbarung die Verwendung eines oben beschriebenen Glases und/oder glaskeramischen und/oder keramischen Materials zur Herstellung eines in den menschlichen oder tierischen Körper einbringbaren, insbesondere eines in den menschlichen oder tierischen Körper implantierbaren, oder zur Herstellung eines an den menschlichen oder tierischen Körper anbringbaren Elements. In einer bevorzugten Ausbildungsform betrifft die Offenbarung eines oben beschriebenen Glases und/oder glaskeramischen Materials zur Herstellung von Implantaten, hermetischen Durchführungen, Gefäßstützen, insbesondere Stents, Kathetern, künstlichen Herzklappen, Gehäusen für elektronische Geräte wie Transponder oder ganze Defibrillatoren, Herzschrittmacher oder Labs-on-Chip, sowie Kontaktlinsen und/oder Beschichtung auf Kontaktlinsen, wobei bei der Verwendung als Kontaktlinse und/oder Beschichtung auf Kontaktlinsen der Gehalt an färbenden Stoffen, z.B. Eisenoxid und weiteren Verunreinigungen so niedrig wie möglich zu wählen ist.

Die Verwendung des Glases als Transpondermaterial hat sich als besonders vorteilhaft gegenüber bekannten Werkstoffen für diese Anwendung erwiesen. Transpondermaterial wird z.B. zur hermetischen Kapselung von RFID-Transpondern verwendet, welche beispielsweise zur Identifikation und Tracking von Haus- und Nutztieren eingesetzt werden. Die Kapselung mit dem beschriebenen Material ermöglicht den Schutz des elektronischen Bauteils innerhalb des Körpers, gleichzeitig schützt sie das umliegende Gewebe gegen die im inneren der Verkapselung befindliche Elektronik. Zudem bietet sie Schutz von Umwelteinflüssen wie Nässe und Schmutz, wenn der Transponder am Körper getragen wird. Hier erweist sich die inhibitorische Wirkung gegen die Bildung von Biofilmen des erfindungsgemäßen Materials ebenso als vorteilhaft. Dies gilt auch für am Körper getragene medizinische Hilfsmittel wie etwa Kontaktlinsen.

Die Transponder können jedoch auch implantiert werden, und bei dieser Anwendung kommt die besonders gute Kompatibilität des erfindungsgemäßen Werkstoffs mit menschlichen beziehungsweise tierischen Zellen zum Tragen. Dies gilt auch für die Verwendung des Glases als Gehäuse oder Gehäusebestandteil für ein anderes elektronisches, medizinisches Gerät, beispielsweise eines aktiven-Implants, und für die Anwendungen als Werkstoff für ein im Körper eingesetztes medizinisches Hilfsmittel wie Implantate, Gefäßstützen, insbesondere Stents, und Katheter, künstliche Herzklappen, Dosiereinrichtungen von Wirkstoffen und dergleichen sowie Kombinationen der genannten Gegenstände.

Das erfindungsgemäße Element eignet sich im Zusammenhang mit allen medizinischen Geräten oder Hilfsmitteln wie Kanülen, Nahtmaterial, auch Klammern, und ähnlichem, welche am oder im Körper angewendet werden.

Die Erfindung wird nachfolgend unter Bezugnahme auf die beigefügten Zeichnungen anhand von Ausführungsbeispielen näher erläutert. Gleiche und ähnliche Bauteile sind dabei mit gleichen Bezugszeichen versehen, wobei die Merkmale der verschiedenen Ausführungsbeispiele miteinander kombiniert und/oder gegeneinander ausgetauscht werden können.

Es zeigen:
- Figur 1: Analyse und Visualisierung des Biofilmwachstums nach Kultivierung über 24 Stunden: Orientierende Datenanalyse mit Biofilm bedeckter Fläche, kumulativ für alle Bakterien,
- Figur 2: Analyse und Visualisierung des Biofilmwachstums nach Kultivierung über 24 Stunden: Quantitative Analyse mit Biofilm bedeckter Fläche gemäß den bakteriellen Spezies,
- Figur 3: Analyse und Visualisierung des Biofilmwachstums nach Kultivierung über 24 Stunden: Repräsentative Aufnahmen von Biofilmen auf Kalk-Natron-Glas,
- Figur 4: Analyse und Visualisierung des Biofilmwachstums nach Kultivierung über 24 Stunden: Repräsentative Aufnahmen von Biofilmen auf Glas gemäß einer ersten Ausführungsform der Erfindung,
- Figur 5: Cytotoxizität und Hämokompatibilität von Kalk-Natron-Glas und verschiedenen Borosilikatgläsern: Repräsentative Aufnahmen kultivierter Muskelzellen, Caco2-Zellen und L929 Maus-Zellen.
- Figur 6: Cytotoxizität und Hämokompatibilität von Kalk-Natron-Glas und verschiedenen Borosilikatgläsern: Daten zur Hämolyse,
- Figur 7: Cytotoxizität und Hämokompatibilität von Kalk-Natron-Glas und verschiedenen Borosilikatgläsern: Daten zur Erythrocytenaggregation,
- Figur 8: Cytotoxizität und Hämokompatibilität von Kalk-Natron-Glas und verschiedenen Borosilikatgläsern: Repräsentative Aufnahmen kultivierter Zellen von MSSA, Enterococcus faecalis, Enterococcus faecium, Escherichia coli, Klebsiella pneumonia, Pseudomonas aeruginosa, Proteus Mirabilis, Staphylococcus epidermis,
- Figur 9: schematische Darstellungen eines implantierbaren Gehäuses für ein elektronisches Bauteil gemäß einer ersten Ausführungsform der Erfindung,
- Figur 10: eine schematische Darstellung einer Gefäßstütze gemäß einer weiteren Ausführungsform der Erfindung.

Um die Hemmung des Biofilmwachstums und das Anwachsen menschlicher sowie tierischer Zellen zu untersuchen, wurden Experimente mit unterschiedlichen Gläsern durchgeführt. Als Ausführungsformen der Erfindung wurden dabei die Gläser "BF1" und "BF2" verwendet. Als Vergleichsbeispiele wurden die Gläser "VG1", "VG2" und "VG3" untersucht.

Die folgende Tabelle 1 gibt einen Überblick über die chemische Zusammensetzung der untersuchten Gläser beziehungsweise Glaskeramiken:

| Anteil (Gew.-%) | VG1 | VG2 | VG3 | BF1 | BF2 |
|---|---|---|---|---|---|
| SiO₂ | 72,48 | 81,19 | 61,59 | 63,03 | 63,06 |
| Al₂O₃ | 1,21 | 2,41 | 16,96 | 4,11 | 4,11 |
| Na₂O | 14,36 | 3,52 | 12,32 | 6,08 | 6,08 |
| K₂O | 1,21 | 0,08 | 4,14 | 8,51 | 8,51 |
| MgO | 4,32 | 0,00 | 3,94 | 0,00 | 0,00 |
| CaO | 6,43 | 0,00 | 0,00 | 0,00 | 0,00 |
| ZnO | 0,00 | 0,00 | 0,00 | 5,83 | 5,83 |
| B₂O₃ | 0,00 | 12,76 | 0,00 | 8,22 | 8,22 |
| CeO₂ | 0,00 | 0,00 | 0,30 | 0,00 | 0,00 |
| TiO₂ | 0,00 | 0,00 | 0,00 | 3,98 | 4,11 |
| SnO₂ | 0,00 | 0,00 | 0,40 | 0,00 | 0,00 |
| Sb₂O₃ | 0,00 | 0,00 | 0,00 | 0,20 | 0,00 |
| F₂ | 0,00 | 0,00 | 0,30 | 0,00 | 0,000 |
| FeO | 0,01 | 0,04 | 0,05 | 0,05 | 0,05 |
| SeO₂ | 0,00 | 0,00 | 0,00 | 0,00 | 0,03 |
| Summe | 100,00 | 100,00 | 100,00 | 100,00 | 100,00 |

Die Gläser wurden in Form von Platten getestet. Die Platten wurden zunächst in 1 % Deconex 12 PA (Borer advanced cleaning solutions) bei 40 °C für 15 min mittels Ultraschall gereinigt. Das Reagens wurde zunächst mit warmem Leitungswasser und dann durch Waschen mit deionisiertem Wasser entfernt. Daraufhin wurden die Platten in deionisiertem Wasser bei 40°C für 5 min mit Ultraschall behandelt. Schließlich wurden die Platten in einem Stickstoffstrom getrocknet.

Für die Tests der Biofilmbildung wurden acht typische Vertreter nosokomialer Infektionen untersucht, nämlich
- MSSA: Staphylococcus aureus, methicillin-sensitiv (ATCC 29213)
- Staphylococcus epidermis (Pp 62a)
- Enterococcus faecalis (ATCC 29212)
- Enterococcus faecium (SMZ 20477)
- Escherichia coli (ATCC 25922)
- Klebsiella pneumonia (ATCC 700603)
- Pseudomonas aeruginosa (PA01)
- Proteus Mirabilis (VBK 4479).

Die Bakterienmutterkulturen wurden bei -80°C in 10 vol.-% Glycerinlösung gelagert. Vor der Kultivierung in flüssigem Medium wurde ein Strang gefrorener Bakterien ausgestrichen auf Lysogenie-Broth (LB) agar (1,5%)-Platten und über Nacht bei 37°C inkubiert. Eine einzelne Kolonie jeder der für den Menschen pathogenen Stämme wurde in Mueller-Hinton (MH)-Medium über Nacht bei 37°C kultiviert.

Zur Biofilmbildung wurden die Kulturen frisch in MH-Medium geimpft in einer Verdünnung von 1:1000 und in 8 x 2 Abschnitte der an den Glasplatten angebrachten Silikonaufbauten (SCHOTT Nexterion) gefüllt. Biofilme wurden bei 37°C über einen Zeitraum von 24 Stunden ohne Schütteln gezogen.

Die Biofilme wurden mit Hilfe des LIVE/DEAD BacLight Bacterial Viability Kit (Life Technologies) angefärbt. Nach dem Anfärben wurde der bakterielle Überstand vorsichtig entfernt und die Biofilme vorsichtig einmal mit 300 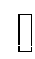 L (Mikroliter) einer 0,9%igen NaCl-Lösung gewaschen . Daraufhin wurden sie in Fluoromount-G (SouthernBiotech, BIOZOL) eingebettet und mit Nexterion(R)-Glas Deckplatten (SCHOTT Nexterion) abgedeckt. Die Visualisierung der Biofilme erfolgte wie oben beschrieben.

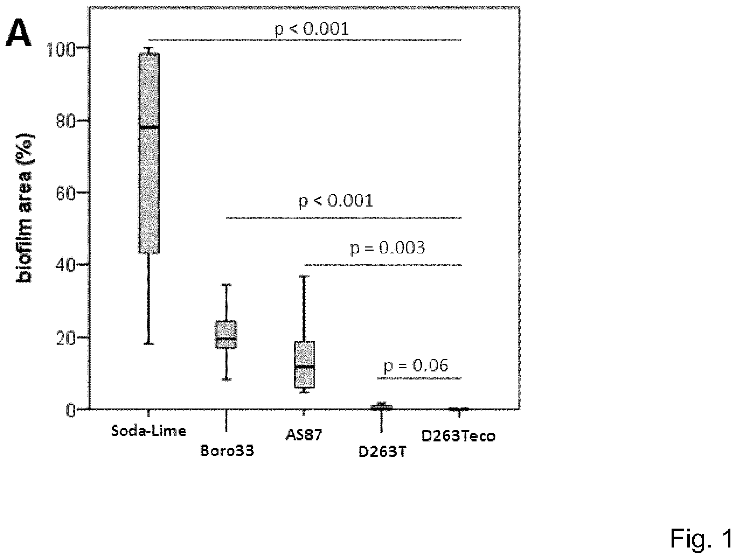

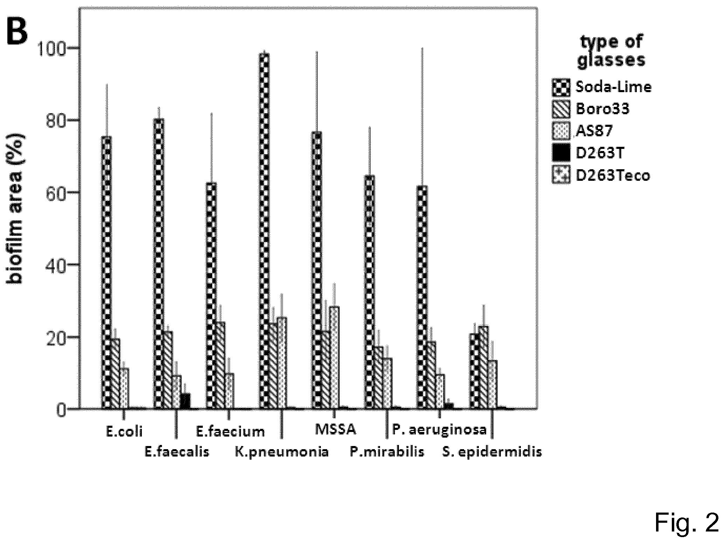

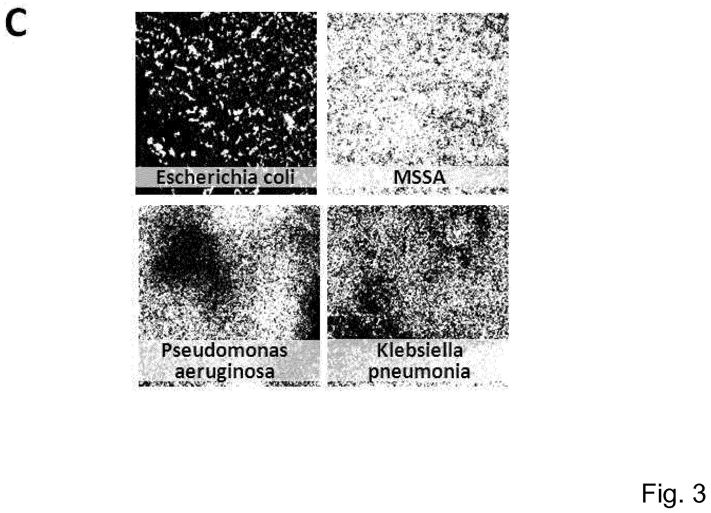

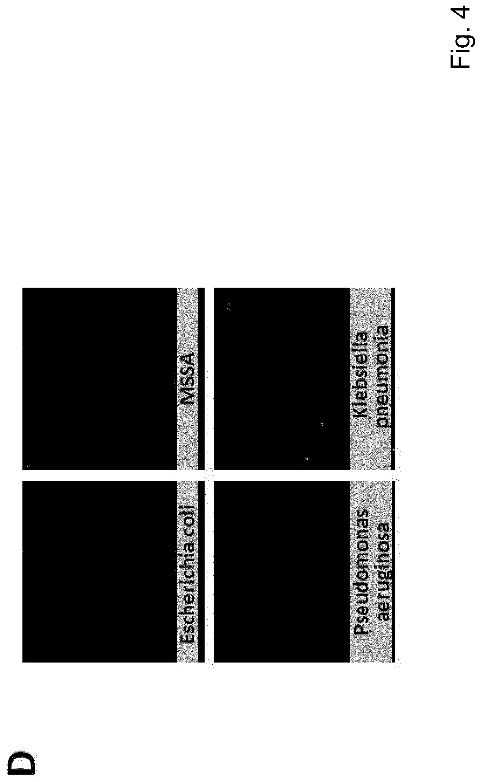

In den Figuren 1 bis 4 sind Ergebnisse der Analyse und Visualisierung des Biofilmwachstums nach Kultivierung über 24 Stunden wiedergegeben. Das Symbol p steht für den "p-Wert" bzw. Signifikanzwert und ist eine Kennzahl zur Auswertung statistischer Tests. In den Biowissenschaften hat sich eine Grenze von 5% etabliert (maximale Irrtumswahrscheinlichkeit oder Signifikanzniveau α = 0,05). Das heißt: Ist die Wahrscheinlichkeit, dass ein Ergebnis zufällig zustande gekommen ist, kleiner als 5%, gilt es als "signifikant" (p < 0,05).

Die statistische Analyse der mit Biofilm bedeckten Flächen (kumulativ für alle Spezies) ist in der folgenden Tabelle 2 wiedergegeben. Darin bezeichnet N die Anzahl der Experimente und SEM den "standard error of mean".

| Glastyp | Biofilmfläche (%) | | Biofilmdicke (um) | Grad der Biofilmhomogenität (% unvollständig) |
|---|---|---|---|---|
| | Mittelwert | SEM | | |
| VG1 (N=24) | 69,33 | 7,041 | 10 bis 30 | <10 |
| VG2 (N=27) | 21,03 | 1,500 | 10 bis 30 | < 20 |
| VG3 (N=25) | 14,32 | 1,875 | 10 bis 30 | < 50 |
| BF1 (N=27) | 0,76 | 0,294 | 4 bis 10 | > 95 |
| BF2 (N=25) | 0,07 | 0,026 | 4 bis 10 | > 95 |

Alle untersuchten humanpathogenen Stämme wuchsen gut auf den Oberflächen des Glases entsprechend VG1 sowie der Platten mit der Zusammensetzung von VG2 und VG3. Nach 24 Stunden lebten die meisten der Bakterien, was mittels grüner Färbung durch SYTO9 nachgewiesen werden kann. Die sporadischen toten Zellen (rot gefärbt durch Propidiumiodid) entsprechen der üblichen Abnahme in Biofilmen.

Auf den Gläsern BF1 und BF2 zeigten alle untersuchten humanpathogenen Keime eine verminderte Bildung von Biofilmen. Die Biofilmdicke ist stark reduziert und liegt im Bereich zwischen 4 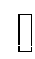 m und 10 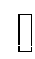 m (Mikrometern). Zudem zeigen die Biofilme auf den erfindungsgemäßen Gläsern morphologische Veränderungen mit diffusen und porösen Strukturen. Die überhaupt von Biofilm bedeckten Flächen von 0,76 +/- 0,294 % beziehungsweise 0,07 +/- 0,026 % sind stark reduziert im Vergleich mit den übrigen untersuchten Gläsern. Zudem zeigen die vorhandenen Biofilme auf den erfindungsgemäßen Gläser eine stark erhöhte Porosität von oberhalb 95 % im Vergleich mit den übrigen untersuchten Gläsern. Ebenso wie die gut gewachsenen Biofilme auf VG1, VG2 und VG3 zeigen die verminderten Biofilme lediglich eine geringe Anzahl toter Zellen.

Diese Ergebnisse legen überraschenderweise nahe, dass die chemische Zusammensetzung der erfindungsgemäßen Materialien BF1 und BF2 den ersten Schritt zur Anhaftung von Bakterien verhindert. Bisher wurden die untersuchten erfindungsgemäßen Gläser als widerstandsfähige Abdeckungen für Touchpanels bei in Automobilen eingebauten Navigationsgeräten oder Substrate für kapazitive Sensoren sowie Filter für Kameramodule in Mobiltelefonen und als Gehäuse für CCD-Bildsensoren eingesetzt. Von daher hatte ein Fachmann auf dem Gebiet der Mikrobiologie mit diesen Gläsern keinerlei Kontakt.

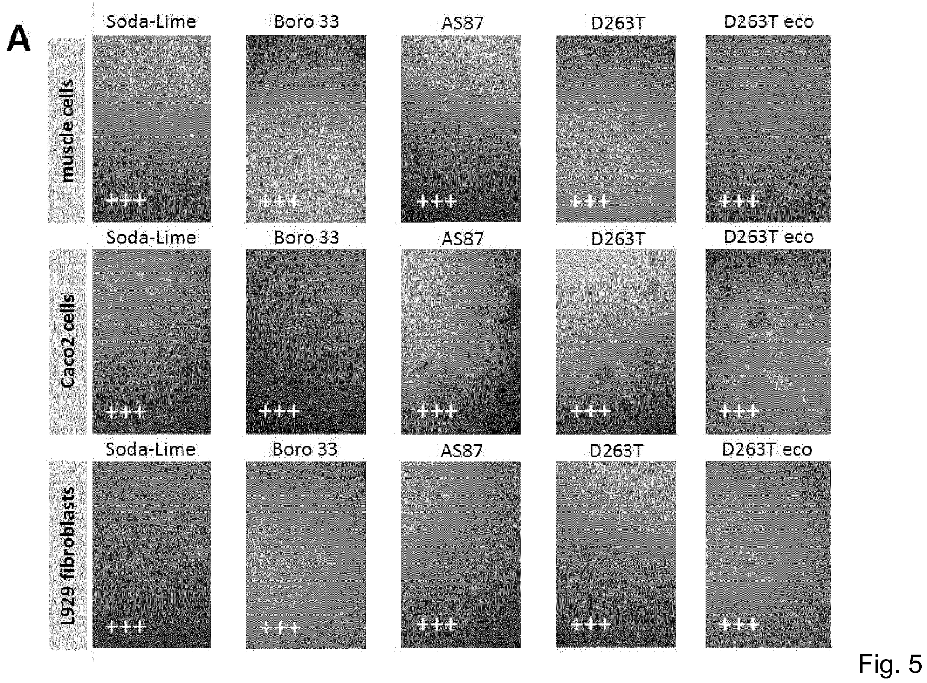

In den Figuren 5 bis 8 sind Ergebnisse zur in vitro bestimmten Cytotoxizität und Hämokompatibilität von Kalk-Natron-Glas und verschiedenen Borosilikatgläsern wiedergegeben.

Um Änderungen der Zellmorphologie und Wachstumsleistung zu bewerten, wurden Muskelzellen, Caco2-Zellen und L929-Mausfibroblasten über 48 Stunden auf den verschiedenen Gläsern kultiviert. Verglichen mit Kontrollzellen in Zellkulturkolben wurden für alle Gläser keine Veränderungen der Zellmorphologie und der Wachstumsleistung gefunden. Wie die Bilder in Figur 5 zeigen, sind keine apoptotischen Zellen und Abnormalitäten in der Zellumgebung zu finden. Keine der untersuchten Glaszusammensetzungen zeigte irgendwelche Anzeichen für Cytotoxizität.

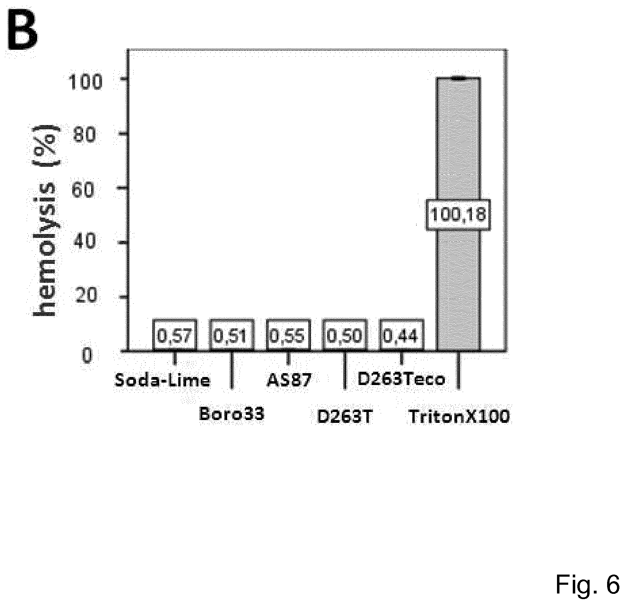

Zur Untersuchung der Hämolyse wurden Erythrocyten auf den Glasoberflächen für zwei Stunden inkubiert. Wie die in Figur 6 aufgetragenen Ergebnisse belegen, zeigt unabhängig von der Glaszusammensetzung keines der Gläser Anteile von Hämolyse von mehr als 0,57 %. Gemäß dem ASTM-Standard F756-00 wird eine Hämoglobinfreisetzung zwischen 0 und 2 % als nicht-hämolytisch angesehen. Dies zeigt, dass keine nachweisbare Störung der Membranen der roten Blutkörperchen vorliegt.

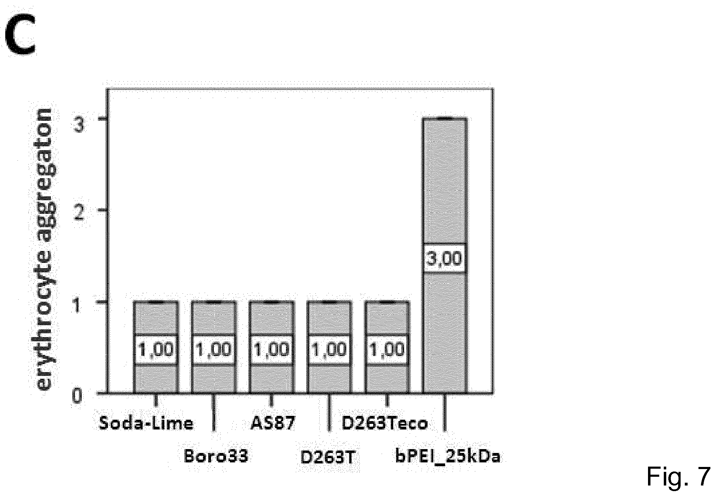

Zusätzlich wurde untersucht, inwieweit die Materialarten geeignet sind, Aggregation von Erythrocyten hervorzurufen. Die Aggregation von Erythrocyten ist ein unerwünschtes Phänomen, welches zu Nebenwirkungen auf den Kreislauf und sogar tödlicher Toxizität führt. Die Aggregation von Erythrocyten wurde mikroskopisch sichtbar gemacht nach zweistündiger Inkubation auf dem Glasoberflächen. Eine Behandlung mit 25 kDa bPEI bei 30 mg/mL als positive Kontrolle führte zur Bildung von Aggregaten (Stufe 3 gemäß der oben erläuterten Einteilung). Wie Figur 7 zeigt, ergab sich aber für keines der untersuchten Materialien eine Aggregation roter Blutkörperchen. Zudem zeigten alle Glaszusammensetzungen eine sehr gute Hämokompatibilität.

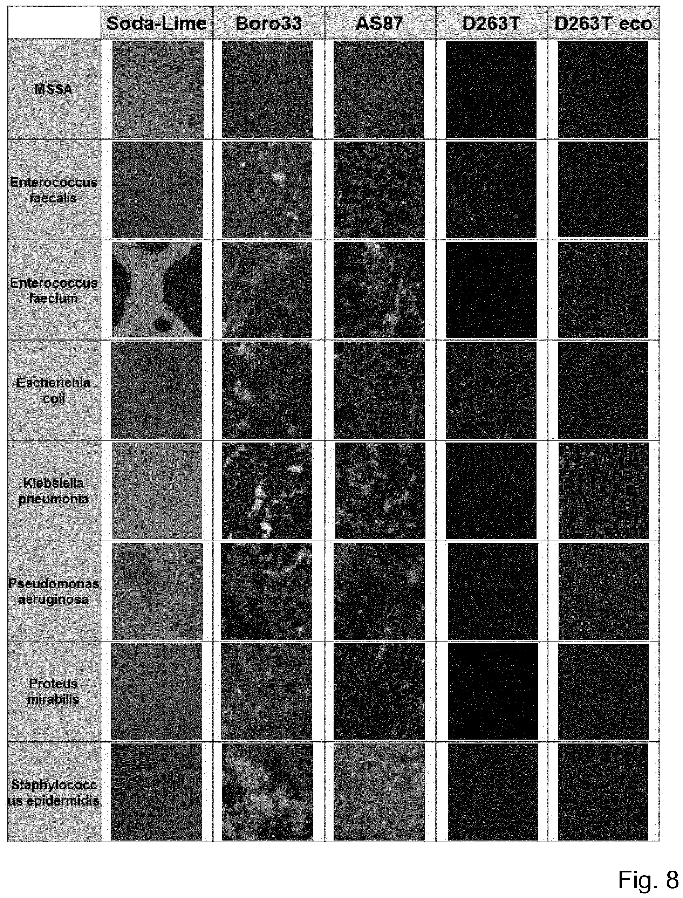

Grundsätzlich zeigte von den verwendeten Keimen S. epidermis eine geringe Neigung zur Bildung von Biofilmen auf den Gläsern. K. pneumonia und MSSA zeigten dagegen eine erhöhte Tendenz zu Adhäsion und Wachstum auf den Glasoberflächen, wie die Aufnahmen in Figur 8 zeigen. Auch für diese Keime ist die Biofilmbildung auf den erfindungsgemäßen Gläsern jedoch deutlich reduziert.

Für die Entwicklung hoch-effizienter anti-biofilm-Oberflächen oder Beschichtungen sind Gläser und/oder Glaskeramiken und/oder Keramiken mit der erfindungsgemäßen Zusammensetzung damit besonders geeignet, das erfindungsgemäße Glas und/oder glaskeramische und/oder keramische Material hat gegenüber den Vergleichbeispielen VG1 bis VG3 eine reduzierte Biofilmanhaftung, eine erhöhte Biofilmporosität und reduzierte Biofilmdicke. Zudem sind sie exzellent cyto- und hämokompatibel ohne toxisch für eukaryotische Zellen zu sein.

Mit diesen überraschenden Eigenschaften ergeben sich im Bereich der Medizin diverse Anwendungsmöglichkeiten für die Erfindung.

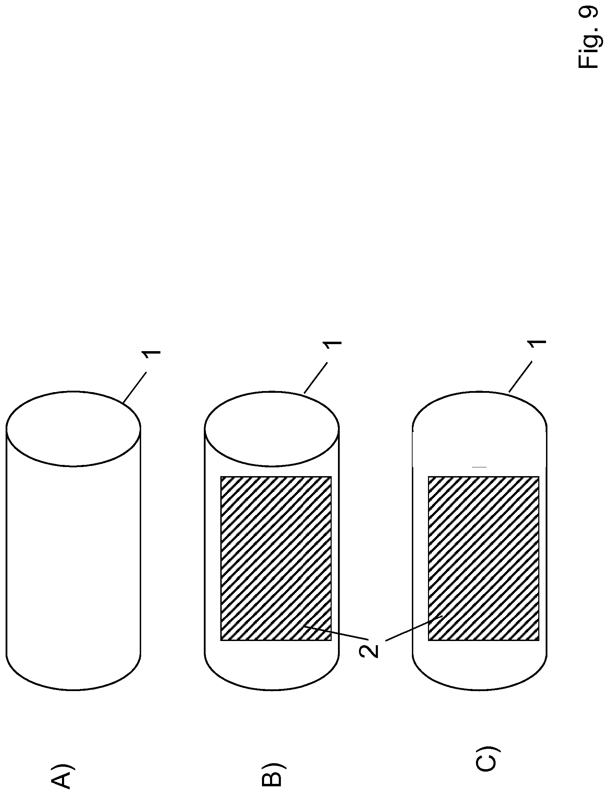

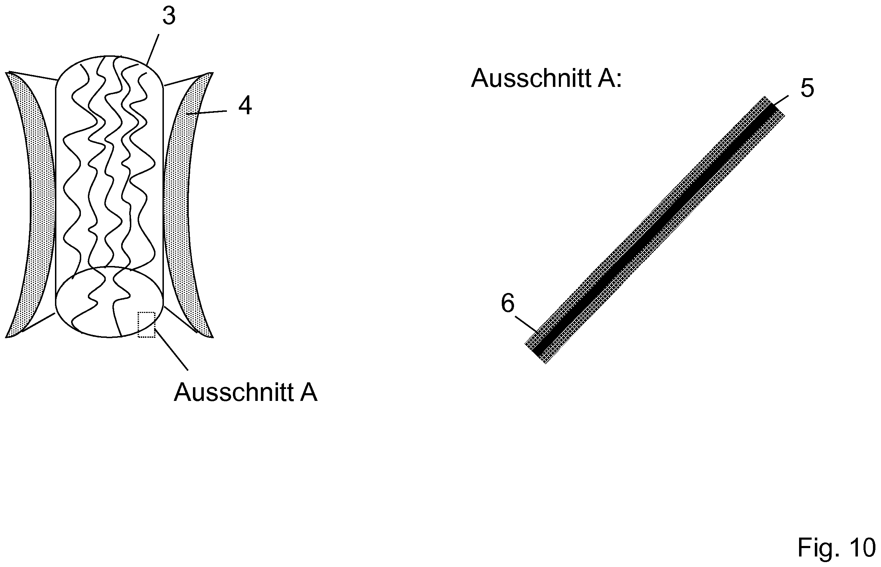

In den Figuren 9 und 10 sind exemplarische Anwendungen für das Glas beziehungsweise für ein glaskeramisches Material dargestellt.

In Figur 9 ist ein Gehäuse 1 für elektronische Bauteile 2 dargestellt. Gehäuse für Transponder, sogenannte Transponderröhrchen, werden in einfacher Weise als Abschnitte von Rohren gefertigt. Beispielsweise wird dann ein Ende durch Zuschmelzen verschlossen. Ein derartiges Gehäuse für einen Transponder ist in Figur 9 oben unter A) dargestellt. Durch das noch offene Ende - in der Abbildung auf der rechten Seite zu sehen - wird dann eine elektronische Komponente, "Transponder" genannt, in das Rohr eingebracht, vergleiche Figur 9 B). Bei dem "Transponder" kann es sich beispielsweise um einen RFID-Chip oder einen Sensor handeln. Unter Anwendung von Hitzeeinwirkung, beispielsweise von Laser- und/oder infraroter Strahlung, kann dann das noch offene Ende des Rohres verschlossen werden. Dann ist das Gehäuse vorteilhafterweise hermetisch geschlossen wie in Figur 9 C) gezeigt. In einer bevorzugten Ausgestaltung der Erfindung ist das Gehäuse autoklavierbar.

Der Anteil von FeO im Glas kann das Verschließen des Rohres insbesondere durch die Anwendung von Laser- und/oder infraroter Strahlung fördern, insbesondere weil dadurch die Absorption des Material im infraroten Spektralbereich erhöht wird.

Typische Abmessungen für derartige Transpondergehäuse liegen bei Außendurchmessern im Bereich von bis zu 10 mm, bevorzugt im Bereich bis 5 mm, besonders bevorzugt im Bereich zwischen 1 mm und 4 mm, und bei Längen im Bereich von bis zu 100 mm, bevorzugt im Bereich von bis zu 70 mm, besonders bevorzugt im Bereich zwischen 5 mm und 50 mm. Die Wanddicke liegt insbesondere im Bereich von bis zu 2 mm, bevorzugt im Bereich von bis zu 1,5 mm, besonders bevorzugt im Bereich zwischen 0,03 mm und 1,1 mm.

Ebenso möglich ist es, dass das beispielsweise als Rohrabschnitt ausgeformte Element auf zumindest einer Seite mit einer für einen Wirkstoff durchlässigen Einrichtung, beispielsweise einer Membran, versehen und der Innenraum mit einem Wirkstoff versehen wird. Auf diese Weise lässt sich auf einfache Weise ein Implantat zur Verabreichung von Wirkstoffen herstellen.

Ebenso möglich ist es, elektronische Elemente in ein solches Implantat zur Verabreichung von Wirkstoffen zu integrieren, wobei das elektronische Element insbesondere die Bedingungen der Wirkstoffabgabe steuern und/oder auslösen kann, insbesondere den Zeitpunkt und/oder die Menge der Wirkstoffabgabe. Dieses elektronische Element kann insbesondere auch so ausgebildet sein, dass es mit elektronischen Einrichtungen außerhalb des Körpers mittels elektrischer Leiter, z.B. Drähten und/oder Kontakten, aber auch durch kabellose Kommunikation kommunizieren kann, welche insbesondere Steuer- und/oder Sensorsignale an das elektronische Element innerhalb des Implantats übermitteln können. So kann das beschriebene Implantat Teil eines komplexeren Diagnose- und/oder Behandlungsapparats sein kann.

Analog ist es möglich, dass durch die durchlässige Einrichtung Substanzen, insbesondere Flüssigkeiten, aus dem menschlichen oder tierischen Körper in das Innere des Implantats gelangen können und dann von dem und/oder den elektronischen Elementen analysiert werden. Aus der Analyse gewonnene Daten können an elektronische Einrichtungen außerhalb des menschlichen oder tierischen Körpers übertragen werden.

In Figur 10 ist ein Stent 3 als eine weitere Anwendungsmöglichkeit des Glases dargestellt. Der Stent 3 ist in ein Hohlorgan 4, beispielsweise ein Blutgefäß, eingesetzt. Der Stent 3 ist aus einem Metallgeflecht, beispielsweise aus Titan, gefertigt. Die Vergrößerung des Abschnittes A auf der rechten Seite der Figur zeigt einen Ausschnitt von einem Metallfilament 5 des Metallgeflechts. Die Metallfilamente 5 weisen gemäß der Erfindung zumindest bereichsweise eine Beschichtung 6 aus dem Glas und/oder glaskeramischen Material gemäß einer oben beschriebenen Zusammensetzung auf. Dadurch wird die Anfälligkeit des Stents 3 für eine Besiedlung gegenüber bekannten Werkstoffen deutlich reduziert. Zudem ist ein derart beschichteter Stent für den menschlichen oder tierischen Körper sehr gut verträglich.

## Patentansprüche

1. In den menschlichen oder tierischen Körper einbringbares Element (1; 3),
wobei das Element aus der Gruppe ausgewählt ist, welche Gehäuse für elektronische Geräte wie Transponder, Defibrillatoren, Herzschrittmacher umfasst, und das Element zumindest bereichsweise ein Glas aufweist,
welches die Bildung von Biofilmen hemmt und/oder nicht cytotoxisch für menschliche oder tierische Zellen und blutverträglich ist, wenn das Element in den menschlichen oder tierischen Körper eingebracht oder an diesen angebracht ist,
wobei das Glas zumindest
| | |
|---|---|
| SiO₂ | im Bereich von 60 bis 75 Gew.-% und |
| ZnO | im Bereich von 1 bis 7 Gew.-% |
umfasst,
**dadurch gekennzeichnet, dass**
das Element zumindest ein Gehäuse (1) umfasst oder ein Gehäuse (1) ist, welches für die Aufnahme elektronischer Bauteile (2) ausgebildet ist,
wobei das Gehäuse zumindest bereichsweise aus dem Glas besteht und/oder
wobei das Gehäuse zumindest bereichsweise zumindest eine Schicht aus dem Glas aufweist.

2. Element (1; 3) nach Anspruch 1,
wobei das Glas zumindest die folgenden Komponenten umfasst
| | |
|---|---|
| SiO₂ | im Bereich von 60 - 75 Gew.-% |
| R₂O | im Bereich von 5 bis 20 Gew.-% |
| RO | im Bereich von 0 bis 10 Gew.-% |
| Al₂O₃ | im Bereich von 1 bis 6 Gew.-% |
| B₂O₃ | im Bereich von 0 bis 10 Gew.-% |
| TiO₂ | im Bereich von 0 bis 8 Gew.-% |
| ZnO | 1 bis 7 Gew.-% |
| FeO | 0 bis 5 Gew.-% |
wobei R₂O ein Oxid oder eine Kombination von Oxiden ist ausgewählt aus der Gruppe, welche Li₂O, Na₂O und K₂O
umfasst,
und wobei RO ein Oxid oder eine Kombination von Oxiden ist ausgewählt aus der Gruppe, welche MgO und/oder CaO und/oder BaO und/oder SrO umfasst.

3. Element (1; 3) nach Anspruch 1 oder 2,
wobei das Glas
| | |
|---|---|
| SiO₂ | im Bereich von 60 bis 70 Gew.-% |
| R₂O | im Bereich von 5 bis 20 Gew.-% |
| RO | im Bereich von 0 bis 10 Gew.-% |
| Al₂O₃ | im Bereich von 1 bis 5 Gew.-% |
| B₂O₃ | im Bereich von 0 bis 10 Gew.-% |
| TiO₂ | im Bereich von 0 bis 8 Gew.-% |
| ZnO | im Bereich von 1 bis 7 Gew.-% |
| FeO | im Bereich von 0 bis 5 Gew.-% |
| | mit |
| Na₂O | im Bereich von 2 bis 10 Gew.-% und/oder |
| K₂O | im Bereich von 3,5 bis 10 Gew.-%. |
| | umfasst, |
wobei R₂O ein Oxid oder eine Kombination von Oxiden ist ausgewählt aus der Gruppe, welche Li₂O, Na₂O und K₂O umfasst,
und wobei RO ein Oxid oder eine Kombination von Oxiden ist ausgewählt aus der Gruppe, welche MgO und/oder CaO und/oder BaO und/oder SrO umfasst

4. Element (1; 3) nach einem der vorangegangenen Ansprüche,
**dadurch gekennzeichnet, dass**
das Glas
einen Anteil von Ag von weniger als 0,3 Gew.-%. und/oder
einen Anteil von P₂O₅ von weniger als 0,5 Gew.-%. und/oder
einen Anteil von F von weniger als 1 Gew.-%. umfasst.

5. Element (1) nach einem der vorstehenden Ansprüche,
**dadurch gekennzeichnet, dass**
das Gehäuse ein Rohr aus dem Glas umfasst oder aus diesem gebildet wird.

6. Element nach einem der Ansprüche 1 bis 4,
wobei das Gehäuse aus Ti und/oder einer Ti-Legierung ausgebildet ist und ein Isolationselement , welches aus dem Glas ausgebildet ist, in einer Durchführungsöffnung des Gehäuses enthält.

## Claims

1. An element (1; 3) introducible into a human or animal body,
wherein the element is selected from the group consisting of casings for electronic devices such as transponders, defibrillators, pacemakers, and wherein the element comprises a glass at least in some areas thereof,
which inhibits the formation of biofilms, and/or which is not cytotoxic to human or animal cells and is compatible with blood, when the element is introduced into the human or animal body or attached thereto;
wherein the glass comprises at least:
SiO₂ in a range from 60 to 75 wt%, and
ZnO in a range from 1 to 7 wt%;
**characterized in that**
the element comprises at least one casing (1) or constitutes a casing (1) adapted for accommodating electronic components (2);
wherein the casing is made of said glass at least in areas thereof; and/or
wherein the casing has at least one coating made of said glass at least in areas thereof.

2. The element (1; 3) according to claim 1,
wherein the glass comprises at least the following components:
| | |
|---|---|
| SiO₂ | in a range from 60 to 75 wt% |
| R₂O | in a range from 5 to 20 wt% |
| RO | in a range from 0 to 10 wt% |
| Al₂O₃ | in a range from 1 to 6 wt% |
| B₂O₃ | in a range from 0 to 10 wt% |
| TiO₂ | in a range from 0 to 8 wt% |
| ZnO | 1 to 7 wt% |
| FeO | 0 to 5 wt%; |
wherein R₂O is an oxide or a combination of oxides selected from the group consisting of Li₂O, Na₂O and K₂O;
and wherein RO is an oxide or a combination of oxides selected from the group consisting of MgO and/or CaO and/or BaO and/or SrO.

3. The element (1; 3) according to claim 1 or 2,
wherein the glass comprises
| | |
|---|---|
| SiO₂ | in a range from 60 to 70 wt% |
| R₂O | in a range from 5 to 20 wt% |
| RO | in a range from 0 to 10 wt% |
| Al₂O₃ | in a range from 1 to 5 wt% |
| B₂O₃ | in a range from 0 to 10 wt% |
| TiO₂ | in a range from 0 to 8 wt% |
| ZnO | in a range from 1 to 7 wt% |
| FeO | in a range from 0 to 5 wt%; |
| with | |
| Na₂O | in a range from 2 to 10 wt%; and/or |
| K₂O | in a range from 3.5 to 10 wt%; |
wherein R₂O is an oxide or a combination of oxides selected from the group consisting of Li₂O, Na₂O and K₂O;
and wherein RO is an oxide or a combination of oxides selected from the group consisting of MgO and/or CaO and/or BaO and/or SrO.

4. The element (1; 3) according to any one of the preceding claims,
**characterized in that** the glass comprises
a content of Ag of less than 0.3 wt%; and/or
a content of P₂O₅ of less than 0.5 wt%; and/or
a content of F of less than 1 wt%.

5. The element (1) according to any one of the preceding claims,
**characterized in that**
the casing comprises or is formed from a tube made of said glass.

6. The element (1) according to any one of claims 1 to 4,
wherein the casing is made from Ti and/or a titanium alloy and comprises an insulating element which is made from said glass in a feedthrough opening of the casing.

## Revendications

1. Élément (1 ; 3) pouvant être introduit dans le corps humain ou animal,
l'élément étant choisi dans le groupe qui comprend des boîtiers destinés à des dispositifs électroniques, tels que des transpondeurs, des défibrillateurs, des stimulateurs cardiaques, et l'élément présentant, au moins dans certaines parties, un verre
qui est inhibiteur de la formation de biofilms et/ou n'est pas cyto-toxique pour des cellules humaines ou animales et est compatible avec le sang lorsque l'élément est introduit dans le corps humain ou animal ou est fixé à celui-ci,
le verre comprenant au moins
SiO₂ dans la plage allant de 60 à 75 % en poids et
ZnO dans la plage allant de 1 à 7 % en poids,
**caractérisé en ce que**
l'élément comprend au moins un boîtier (1) ou est un boîtier (1) qui est conçu pour accueillir des composants électroniques (2),
le boîtier étant constitué du verre, au moins en partie, et/ou
le boîtier présentant au moins une couche du verre, au moins dans certaines parties.

2. Élément (1 ; 3) selon la revendication 1,
dans lequel le verre comprend au moins les constituants suivants :
| | |
|---|---|
| SiO₂ | dans la plage allant de 60 à 75 % en poids |
| R₂O | dans la plage allant de 5 à 20 % en poids |
| RO | dans la plage allant de 0 à 10 % en poids |
| Al₂O₃ | dans la plage allant de 1 à 6 % en poids |
| B₂O₃ | dans la plage allant de 0 à 10 % en poids |
| TiO₂ | dans la plage allant de 0 à 8 % en poids |
| ZnO | 1 à 7 % en poids |
| FeO | 0 à 5 % en poids, |
où R₂O est un oxyde ou une combinaison d'oxydes, choisis dans le groupe comprenant Li₂O, Na₂O et K₂O,
et où RO est un oxyde ou une combinaison d'oxydes, choisis dans le groupe comprenant MgO et/ou CaO et/ou BaO et/ou SrO.

3. Élément (1 ; 3) selon la revendication 1 ou 2,
dans lequel le verre comprend du
| | |
|---|---|
| SiO₂ | dans la plage allant de 60 à 70 % en poids |
| R₂O | dans la plage allant de 5 à 20 % en poids |
| RO | dans la plage allant de 0 à 10 % en poids |
| Al₂O₃ | dans la plage allant de 1 à 5 % en poids |
| B₂O₃ | dans la plage allant de 0 à 10 % en poids |
| TiO₂ | dans la plage allant de 0 à 8 % en poids |
| ZnO | dans la plage allant de 1 à 7 % en poids |
| FeO | dans la plage allant de 0 à 5 % en poids |
| avec du | |
| Na₂O | dans la plage allant de 2 à 10 % en poids et/ou |
| K₂O | dans la plage allant de 3,5 à 10 % en poids, |
où R₂O est un oxyde ou une combinaison d'oxydes, choisis dans le groupe comprenant Li₂O, Na₂O et K₂O,
et où RO est un oxyde ou une combinaison d'oxydes, choisis dans le groupe comprenant MgO et/ou CaO et/ou BaO et/ou SrO.

4. Élément (1 ; 3) selon une des revendications précédentes,
**caractérisé en ce que** le verre comprend
une proportion d'Ag inférieure à 0,3 % en poids et/ou
une proportion de P₂O₅ inférieure à 0,5 % en poids et/ou
une proportion de F inférieure à 1 % en poids.

5. Élément (1) selon une des revendications précédentes,
**caractérisé en ce que** le boîtier comprend un tube, constitué du verre, ou est réalisé à partir de celui-ci.

6. Élément (1) selon une des revendications 1 à 4, dans lequel le boîtier est réalisé à partir de Ti et/ou d'un alliage de Ti et comporte un élément isolant réalisé à partir du verre, dans une ouverture de passage du boîtier.
